# EUROPEAN PATENT APPLICATION

(11) **EP 4 582 434 A1**
(43) Date of publication of application: **09.07.2025**
(21) Application number: 23881390.1
(22) Date of filing: 14.08.2023
(51) Int. Cl.: C07H 5/06, C07H 13/04, C07H 13/06, C12Q 1/02

(54) **NON-NATURAL SUGAR, AND SYNTHESIS METHOD THEREFOR AND USE THEREOF**

(30) Priority: 27.10.2022 CN 202211329008
(71) Applicant: Beijing Linxcell Biotechnologies Co., Ltd., Beijing 102600 (CN)
(72) Inventor: CHEN, Xing, Beijing 100871 (CN); CHENG, Bo, Beijing 100871 (CN)
(74) Representative: Hoefer & Partner Patentanwälte mbB
(86) International application number: PCT/CN2023/112917
(87) International publication number: WO 2024/087806

(57) **Abstract**

Disclosed in the present invention are an unnatural sugar, and a synthesis method therefor and the use thereof. The synthesis method comprises: subjecting hydroxyl groups of an amino sugar to per-trimethylsilane protection at room temperature, selectively exposing amino groups on the sugar, and coupling and converting the amino groups at room temperature to obtain a per-trimethylsilane-protected unnatural sugar with orthogonal groups; and removing trimethylsilane protecting groups from the per-trimethylsilane-protected unnatural sugar with orthogonal groups to obtain an unnatural sugar without protecting groups. The present invention takes the advantages of existing unnatural sugars into consideration, such that not only is it ensured that the unnatural sugar can be efficiently utilized by cells, but an S side reaction of the unnatural sugar with cysteine in protein during the metabolic process thereof is also effectively avoided; in addition, efficient metabolic marking is achieved. In a cell test, the usage concentration of a 1,6-bis-acylated unnatural sugar is one order of magnitude lower than that of the unnatural sugar without protecting groups.

## Description

### Field

The present invention relates to the technical field of organic chemical synthesis, and in particular to an unnatural sugar and synthesis method therefor and use thereof.

### Background

Glycosylation modification has important functions, and observation of the glycosylation modification is of great significance. In cells, metabolic labeling of unnatural sugars with chemical reporters has been widely used for glucose imaging and glycoproteome analysis. In vitro, chemoenzymatic methods add the unnatural sugars with chemical reporters to carbohydrate chain under the action of glycosyltransferases for further study.

Unnatural sugars are necessary for metabolic labeling and chemoenzymatic labelling and can be divided into three categories.
a. Unnatural sugars without protecting groups: can be used for metabolic labelling, but with high concentration requirements, and are also synthetic substrates for unnatural nucleoside sugars used in chemoenzymatic methods.
b. Per-O-acetylated sugars: developed to overcome the poor cell permeability of unnatural sugars without protecting groups, significantly reducing the concentration required for metabolic labeling. Currently, the per-O-acetylated unnatural sugar probes have been commercialized and sold by companies such as Sigma-Aldrich and Click Chemical Tools.
   However, in 2018, people discovered for the first time that per-O-acetylated sugars undergo non-enzyme-catalyzed S-glyco modification with thiols of protein cysteine, introducing false positive signals for applications such as imaging and mass spectrometry identification.
c. Partially protected unnatural sugars: After the discovery of the S-glyco modification, unnatural sugars with partial hydroxyl acylation came into being. They can easily pass through the cell membrane to achieve efficient metabolic glycan labeling without introducing the S-glyco modification.

Currently, there are mainly two methods for preparing unnatural sugars containing azide at N-acyl position and unprotected hydroxyl groups. Method 1 is to first synthesize sugar derivatives with halogen at N-acyl position, and then introduce azide into the N-acyl position of the sugar by replacing the halogen with the azide; and method 2 is to first synthesize azidoacetic acid, and then couple the azidoacetic acid and amino sugar to obtain unnatural sugars with azide at the N-acyl position. Unnatural sugars containing other orthogonal groups (such as alkynyl) and unprotected hydroxyl groups are synthesized using the strategy of directly coupling amino sugars and small molecules containing orthogonal groups in the above method 2.

However, both of these synthesis methods have disadvantages. In method 1, sodium azide is in large excess and the reaction requires heating, which poses a risk of explosion; and in method 2, the synthesis of azidoacetic acid uses iodoacetic acid and excess sodium azide as raw materials, and in order to increase the yield of the azidoacetic acid, aqueous solution containing excess sodium azide needs to be acidified and the azidoacetic acid needs to be extracted with an organic solvent, which poses a risk of explosion. Moreover, the azidoacetic acid has a low boiling point and is volatile, requiring careful handling and can only be synthesized in small quantities. Unprotected unnatural sugars have high polarity, and impurities and products often co-elute when using a silica gel column for chromatographic purification. Therefore, repeated column chromatography purification is required to obtain sufficiently pure unprotected sugars. There are a few reports of using P-2 gel columns to purify unprotected sugars, but the operation is time-consuming, the gel column is expensive and can only purify small amounts of products.

Currently, partially protected unnatural sugars are divided into two categories: 1,3-bis-acylated unnatural sugars and 1,6-bis-acylated unnatural sugars. There are many reported examples of 1,3-bis-acylated unnatural sugars, but the only example of 1,6-bis-acylated unnatural sugars is 1,6-Pr₂GalNAz. The synthesis of 1,3-bis-acylated unnatural sugars is to first synthesize unnatural sugars without protecting groups, and use them as starting materials to obtain the 1,3-bis-acylated unnatural sugars by protecting positions 4 and 6 of the sugar with propylidene, acylating the hydroxyl groups at positions 1 and 3, and finally removing the propylidene protection. In this synthetic route, when synthesizing the unnatural sugars raw materials without protecting groups, the above-mentioned problems are encountered, and when synthesizing the 1,3-bis-acylated unnatural sugars from the unnatural sugars without protecting groups, the overall yield is low. When synthesizing 1,6-Pr₂GalNAz, azidoacetic acid is required as raw materials, and the synthesis of the azidoacetic acid faces the problems stated above.

At present, there are two prominent problems with the unnatural sugars used in the field of chemoenzymatic labeling and metabolic glycan labeling: one is that some unnatural sugars used (such as per-O-acetylated sugars) may introduce false positive results in metabolic labeling, requiring optimization of the sugar structure, and research on partially protected sugars is just beginning and urgently needs to be promoted; the other is that the existing methods for synthesizing these unnatural sugars have many drawbacks, including safety, yield, and high difficulty in large-scale preparation.

Regarding the first type of problem, all per-O-acetylated unnatural sugars may introduce S-glyco modification when used for metabolic labeling. It has been reported in the literature that partially acylated unnatural sugars can avoid S-glyco modification and improve the efficiency of unnatural sugars passing through the cell membrane and entering the cell. **In** the publicly reported materials, these partially acylated unnatural sugars include 1,3-Ac₂GalNAz, 1,3-Pr₂GalNAz, 1,3-Pr₂ManNAz, 1,3-Pr₂GlcNAz, 1,3-Pr₂GlcNAz and 1,6-Pr₂GalNAz. Other novel partially acylated sugars that are of great value in metabolic glycan labeling have not been reported and it is necessary to develop their synthetic methods and carry out their application research.

Regarding the second type of problem, existing methods for synthesizing unnatural sugars have many shortcomings, and it is necessary to develop new synthesis methods to synthesize unnatural sugars of important value in a more economical and efficient way. Taking the synthesis of unnatural sugars containing azide as an example, a large excess of sodium azide needs to be used in the synthesis process, which poses a risk of explosion; the reaction requires heating, which is cumbersome and unsafe; the product of the reaction need to be repeatedly purified through a column, which is costly and difficult to carry out large-scale synthesis.

The above two types of problems seriously restrict the application research related to the unnatural sugars. Therefore, new strategies need to be developed to solve the above two bottleneck problems.

### Summary

The main purpose of the present invention is to provide an unnatural sugar and synthesis method therefor and use thereof, so as to solve the technical problems of the prior art in the synthesis and application of the unnatural sugars.

According to an aspect of the present invention, a method for synthesizing an unnatural sugar comprises: subjecting hydroxyl groups of an amino sugar to per-trimethylsilane protection at room temperature, selectively exposing amino groups on the sugar, and coupling and converting the amino groups at room temperature to obtain a per-trimethylsilane-protected unnatural sugar with orthogonal groups.

Further, trimethylsilane protecting groups are removed from the per-trimethylsilane-protected unnatural sugar with orthogonal groups to obtain an unnatural sugar without protecting groups.

Further, the unnatural sugar without protecting groups is any one of GalNAz, GlcNAz, ManNAz, GalNAl, GlcNAl and ManNAl;
wherein, during the synthesis of GalNAz, GlcNAz, and ManNAz, the trimethylsilane protecting groups are removed by using a hydrogen ion exchange resin, and a final product is precipitated from a solvent; and
during the synthesis of ManNAl, the trimethylsilane protecting groups are removed by silica gel column chromatography purification.

Further, the hydroxyl groups at positions 1 and 6 of the per-trimethylsilane-protected unnatural sugar with orthogonal groups are protected by hydrophobic groups to obtain a partially acylated unnatural sugar.

Further, the hydrophobic groups are any one of acetyl, propionyl, butyryl and valeryl.

Further, for galactose-type and glucose-type per-trimethylsilane-protected unnatural sugar with orthogonal groups, the trimethylsilane protecting groups at positions 1 and 6 of the per-trimethylsilane-protected unnatural sugar are exchanged into corresponding ester bonds by using pyridine as a solvent and adding four equivalents of carboxylic acid and a large excess of corresponding acid anhydride, and the trimethylsilane protecting groups at positions 3 and 4 are removed by using a hydrogen ion exchange resin to obtain a 1,6-bis-acylated unnatural sugar; and
for a mannose-type per-trimethylsilane-protected unnatural sugar with orthogonal groups, the trimethylsilane protecting groups at positions 1 and 6 are selectively and simultaneously removed by adding two equivalents of ammonium acetate to a mixed solvent of dichloromethane and methanol, and positions 1 and 6 are protected with ester bonds in pyridine and the trimethylsilane protecting groups at positions 3 and 4 of the sugar are then removed to obtain a 1,6-bis-acylated mannose-type unnatural sugar derivative.

Further, the orthogonal groups of the per-trimethylsilane-protected unnatural sugar with orthogonal groups are any one of azide groups, terminal alkyne groups, terminal alkene groups, cyclopropene groups, trans-cyclooctene groups, or cyclooctyne groups.

According to another aspect of the present invention, a partially acylated unnatural sugar for metabolic labeling is any one of 1,6-Ac₂GalNAz, 1,6-Pr₂GalNAz, 1,6-Bu₂GalNAz, 1,6-Ac₂GlcNAz, 1,6-Pr₂GlcNAz, 1,6-Bu₂GlcNAz, 1,6-Ac₂ManNAz, 1,6-Pr₂ManNAz, 1,6-Bu₂ManNAz, 1,6-Pr₂ManNAI and 1,6-Pr₂ManNProc;
preferably, the partially acylated unnatural sugar for metabolic labeling is prepared by the above-mentioned method.

The present invention also discloses a metabolic glycan labeling reagent kit comprising the above-mentioned partially acylated unnatural sugar.

The present invention also discloses the use of the above-mentioned partially acylated unnatural sugar in a metabolic glycan labeling reagent kit for any one of HeLa cells, 3T3 cells, CHO cells and MCF-7 cells.

By adopting the above technical solution, the present invention has at least the following beneficial effects:
The unnatural sugar and synthesis method therefor and use thereof provided by the present invention provide a method for large-scale (on the order of 10 grams) and high-efficiency synthesis of unnatural sugars without protecting groups, which has mid reaction conditions, simple operation, and does not require chromatographic column purification. These unnatural sugars include unnatural sugars without protecting groups and unnatural sugars with some hydroxyl groups protected. The unnatural sugars with some hydroxyl groups protected disclosed in the present invention take the advantages of existing unnatural sugars into consideration, ensuring that the unnatural sugars can be efficiently utilized by cells, effectively avoiding S side reaction of unnatural sugar with cysteine in proteins during metabolism process thereof, and achieving efficient metabolic labeling. In cell tests, the usage concentration of a 1,6-bis-acylated unnatural sugar is one order of magnitude lower than that of the unnatural sugar without protecting groups.

### Brief Description of the Drawings

In order to more clearly illustrate the embodiments of the present invention or the technical solutions in the prior art, the drawings required for the embodiments or prior art description will be briefly introduced below. Obviously, the drawings described below are only some embodiments of the present invention. Other drawings can be obtained by ordinary technicians in this field without paying creative work based on these drawings.
Figure 1 shows the reaction results of incubating 1,6-bis-acylated sugars according to embodiments of the present invention with cell lysates or single proteins of HeLa cells;
Figure 2 shows the results of efficient metabolic labeling of 1,6-bis-acylated sugars according to embodiments of the present invention in HeLa cells;
Figure 3 shows the efficient metabolic labeling of 1,6-bis-acylated sugars according to embodiments of the present invention in different cells; and
Figure 4 shows the efficient metabolic labeling of 1,6-Pr₂GalNAz and 1,6-Pr₂ManNAz according to embodiments of the present invention in vivo in mice.

### Detailed Description

In order to clarify the objectives, technical solutions and advantages of the present invention, the embodiments of the present invention are further described in detail below in combination with specific embodiments and with reference to the accompanying drawings.

It should be noted that all expressions of "first" and "second" in the embodiments of the present invention are used to distinguish two non-identical entities or parameters with the same name. It can be seen that "first" and "second" are only for the convenience of expression and should not be understood as limitations on the embodiments of the present invention. The subsequent embodiments will not explain this one by one.

The embodiment of the present invention discloses a method for synthesizing an unnatural sugar, comprising: subjecting hydroxyl groups of an amino sugar to per-trimethylsilane protection at room temperature, selectively exposing amino groups on the sugars, and coupling and converting the amino groups at room temperature to obtain a per-trimethylsilane-protected unnatural sugar with orthogonal groups. The orthogonal groups of the per-trimethylsilane-protected unnatural sugar with orthogonal groups are any one of azide groups, terminal alkyne groups, terminal alkene groups, cyclopropene groups, trans-cyclooctene groups, or cyclooctyne groups. Trimethylsilane protecting groups are removed from the above-mentioned per-trimethylsilane-protected unnatural sugar with orthogonal groups to obtain an unnatural sugar without protecting groups. Wherein, the unnatural sugar without protecting groups can be any one of GalNAz, GlcNAz, ManNAz, GalNAl, GlcNAl and ManNAl; during the synthesis of GalNAz, GlcNAz and ManNAz, the trimethylsilane protecting groups are removed by using a hydrogen ion exchange resin, and a final product is precipitated from a solvent; and during the synthesis of ManNAl, the trimethylsilane protecting groups are removed by silica gel column chromatography purification.

In some embodiments of the present invention, the hydroxyl groups at positions 1 and 6 of the above-mentioned per-trimethylsilane-protected unnatural sugar with orthogonal groups are protected by hydrophobic groups to obtain a partially acylated unnatural sugar. The hydrophobic groups may be any one of acetyl, propionyl, butyryl, and valeryl. For galactose-type and glucose-type per-trimethylsilane-protected unnatural sugars with orthogonal groups, the trimethylsilane protecting groups at positions 1 and 6 of the per-trimethylsilane-protected unnatural sugar are exchanged into corresponding ester bonds by using pyridine as a solvent and adding four equivalents of carboxylic acid and a large excess of corresponding acid anhydride, and the trimethylsilane protecting groups at positions 3 and 4 are removed by using a hydrogen ion exchange resin to obtain a 1,6-bis-acylated unnatural sugar; and for a mannose-type per-trimethylsilane-protected unnatural sugar with orthogonal groups, the trimethylsilane protecting groups at positions 1 and 6 are selectively and simultaneously removed by adding two equivalents of ammonium acetate to a mixed solvent of dichloromethane and methanol, and positions 1 and 6 are protected with ester bonds in pyridine and the trimethylsilane protecting groups at positions 3 and 4 of the sugar are then removed to obtain a 1,6-bis-acylated mannose-type unnatural sugar derivative(which also belong to unnatural sugars).

Some embodiments of the present invention also disclose a partially acylated unnatural sugar for metabolic labeling, wherein the unnatural sugar is a hexose and a pyranose structure, and has acyl modifications on the hydroxyl groups at positions 1 and 2 of the sugar. The unnatural sugar is an unnatural sugar in which 1-hydroxyl group and 6-hydroxyl group are protected by the hydrophobic groups, wherein the unnatural sugar is generally derivatives of hexose with partially protected hydroxyl groups. The hydrophobic groups are acetyl, propionyl, butyryl or valeryl. The partially acylated unnatural sugar for metabolic labeling is prepared by the above-mentioned synthesis method, that is, subjecting the hydroxyl groups of the sugar to per-trimethylsilane (TMS) protection, selectively exposing amino groups on the sugar, and coupling and chemically converting the amino groups to obtain a per-TMS-protected unnatural sugar with orthogonal groups. Starting from the per-TMS-protected with orthogonal groups, the unnatural sugar without protecting groups and partially acylated unnatural sugar can be efficiently synthesized. The unnatural sugar without protecting groups is GalNAz, GlcNAz, ManNAz, ManNAl and ManNProc. The partially acylated unnatural sugar is any one of 1,6-Ac₂GalNAz, 1,6-Pr₂GalNAz, 1,6-Bu₂GalNAz, 1,6-Ac₂GlcNAz, 1,6-Pr₂GlcNAz, 1,6-Bu₂GlcNAz, 1,6-Ac₂ManNAz, 1,6-Pr₂ManNAz, 1,6-Bu₂ManNAz, 1,6-Pr₂ManNAI and 1,6-Pr₂ManNProc.

Some embodiments of the present invention also disclose a metabolic glycan labeling reagent kit, comprising the above-mentioned partially acylated unnatural sugar. The hydroxyl groups at positions 1 and 6 of the partially acylated unnatural sugar are protected. The partially acylated unnatural sugar is a hexose analogue with positions 1 and 6 protected. The partially acylated unnatural sugar is an unnatural sugar in which the hydroxyl groups at positions 1 and 6 are protected by hydrophobic groups. It can achieve metabolic glycan labeling in any of HeLa cells, 3T3 cells, CHO cells and MCF-7 cells.

The above-mentioned embodiments of the present invention are based on the strategy of per-TMS (trimethylsilane) protection, and develop a method for large-scale and high-efficiency synthesis of unnatural sugars without protecting groups (target compound category 1) and high-efficiency synthesis of 1,6-bis-acylated unnatural sugars (target compound category 2).

In general, the embodiments of the present invention start from the amino sugar containing six carbon atoms, protect all hydroxyl groups on the sugar with TMS, selectively expose the amino groups on the sugar, and further couple and convert the amino groups on the sugar, to obtain the per-TMS-protected unnatural sugar with orthogonal groups. The selective exposure of the amino groups by the per-TMS protection method on the one hand can avoid the involvement or interference of hydroxyl groups on the sugar in subsequent coupling reactions when TMS protection is not performed. On the other hand, during the coupling, the reaction can be carried out in a solvent without hydroxyl groups, avoiding the possibility of the hydroxyl groups on the solvent participating in or interfering with the coupling reaction (when the hydroxyl groups are not protected by TMS, the amino sugar can only be dissolved in solvents with hydroxyl groups, such as water and methanol). Therefore, in the per-TMS protection method, the amount of the molecule required for coupling with the amino groups on the sugar is small (one or slightly more than one equivalent), the coupling efficiency is high (almost quantitative reaction) and the solvent used is easy to be evaporated under reduced pressure (generally dichloromethane is used as a coupling solvent). The per-TMS-protected unnatural sugar with orthogonal groups is an important intermediate for synthesizing target compound category 1 and target compound category 2 in this patent.

The per-TMS-protected unnatural sugar obtained by the above-mentioned method does not require column chromatography purification, and the TMS protecting groups can be removed by the hydrogen ion exchange resin to obtain the unnatural sugar without protecting groups (target compound category 1, GalNAz, GlcNAz, ManNAz and ManNAI have been successfully synthesized in large quantities). It should be noted that when this synthesis method is used to synthesize GalNAz, GlcNAz and ManNAz, there is no intermediate chromatographic purification step, and the final product is precipitated from ethanol. When synthesizing ManNAI, the method of precipitation cannot be used, but it can be quickly purified by a single silica gel column chromatography.

In addition, the per-TMS-protected unnatural sugar obtained by the above method can be purified by a simple silica gel column and then undergo two simple reactions to obtain a 1,6-bis-acylated unnatural sugar. Two technical routes have been developed for different sugars.

For galactose-type and glucose-type per-TMS-protected unnatural sugars, the TMS at positions 1 and 6 of the per-TMS-protected unnatural sugars are exchanged into corresponding ester bonds by using pyridine as a solvent and adding four equivalents of carboxylic acid and a large excess of corresponding acid anhydride, and the TMS protecting groups at positions 3 and 4 are removed by using a hydrogen ion exchange resin to obtain a 1,6-bis-acylated unnatural sugar.

For a mannose-type per-TMS-protected unnatural sugar, the TMS protecting groups at positions 1 and 6 of the sugar are selectively and simultaneously removed by adding two equivalents of ammonium acetate to a mixed solvent of dichloromethane and methanol, and positions 1 and 6 are protected with ester bonds in pyridine and the TMS protecting groups at positions 3 and 4 of the sugar are then removed to obtain a 1,6-bis-acylated mannose-type unnatural sugar derivative.

Based on the above synthetic strategy, 1,6-bis-acylated unnatural sugars (target compound category 2) including 1,6-Ac₂GalNAz, 1,6-Pr₂GalNAz, 1,6-Bu₂GalNAz, 1,6-Ac₂GlcNAz, 1,6-Pr₂GlcNAz, 1,6-Bu₂GlcNAz, 1,6-Ac₂ManNAz, 1,6-Pr₂ManNAz, 1,6-Bu₂ManNAz, 1,6-Pr₂ManNAI and 1,6-Pr₂ManNProc can be synthesized efficiently.

It should be pointed out that some literature has synthesized 1,6-Pr₂GalNAz starting from per-TMS-protected GalNAz, however, this method requires the use of azidoacetic acid as a raw material for the synthesis of per-TMS-protected GalNAz. As mentioned above, the synthesis of azidoacetic acid involves the risk of explosion. The synthesis method disclosed in the embodiments of the present invention avoids the problem of synthesizing azidoacetic acid and can be systematically extended to other sugars.

From a technical perspective, compared with existing synthesis methods, the advantages of the synthesis method disclosed in the embodiments of the present invention are that the per-TMS-protected sugar derivative have lower polarity and good solubility in many low-polarity solvents (such as dimethylformamide, dichloromethane, ethyl acetate, petroleum ether, acetonitrile, etc.), and various chemical transformations (such as replacing halogen with azide) can be easily performed to obtain various per-TMS-protected unnatural sugars with orthogonal groups. **In** the above synthesis method, all reactions are performed at room temperature, and there is no chromatographic purification process, large-scale synthesis (on the order of 10 grams) can be carried out. **In** the process of synthesizing unnatural sugars containing azide, only one equivalent of sodium azide is needed, which is small in amount and safe to operate.

From an application perspective, the synthesized 1,6-bis-acylated sugar can easily pass through the cell membrane and enter the cell. In metabolic glycan labeling experiments, S-glyco modification can be effectively avoided and a high metabolic efficiency can be maintained.

In summary, the main purpose of the present invention is to provide a method for large-scale and high-efficiency synthesis of unnatural sugars, and to carry out research on its application. The 1,6-bis-acylated unnatural sugars newly reported in the present invention can improve the efficiency of metabolic glycan labeling without introducing S-glyco modification.

It should be noted that the embodiments in the present application and features in the embodiments may be combined with each other without conflict.

Unnatural sugar: chemical modification of natural monosaccharide, attaching biological orthogonal groups such as azide and alkyne. The unnatural sugar can be taken up by cells, incorporated into carbohydrate chain via native metabolic glycan pathways, and then glycans can be labeled, imaged, or enriched via bioorthogonal reactions.

Bioorthogonal reaction: refers to chemical reactions that can be carried out in living cells or tissues without interfering with the biochemical reactions of the organism itself, used to study biological macromolecules such as nucleic acids, proteins, sugars or lipids.

The present invention solves the problem of chemical synthesis of unnatural sugars reported in sugar chemical enzymatic labeling and metabolic labeling, and discloses a 1,6-bis-acylated unnatural sugars with novel structures.

The present application studies and improves the existing methods for synthesizing unnatural sugars, develops a strategy based on per-TMS protection, and develops a method for large-scale synthesis of unnatural sugars without protecting groups; at the same time, 1,6-bis-acylated unnatural sugars can be efficiently synthesized starting from the per-TMS-protected unnatural sugars. The 1,6-bis-acylated unnatural sugar can easily pass through the cell membrane and enter the cell. In metabolic glycan labeling experiments, S-glyco modification can be effectively avoid and a high metabolic efficiency can be maintained.

Specifically, the present application starts from the amino sugar containing six carbon atoms, protects all hydroxyl groups on the sugar with TMS, selectively exposes the amino groups on the sugar, and couples the amino groups with various small molecules, to obtain various per-TMS-protected acyl-substituted amino sugar derivatives. The per-TMS-protected sugar derivative has a relatively low polarity and has a good solubility in many low-polarity solvents (such as dimethylformamide, dichloromethane, ethyl acetate, petroleum ether, acetonitrile, etc.), and can undergo various chemical transformations (such as replacing halogen with azide) to obtain various per-TMS-protected unnatural sugars (the per-TMS-protected unnatural sugars contain orthogonal groups such as azide or alkynyl). In methanol, the TMS protecting groups are removed by the hydrogen ion exchange resin. After filtration and concentration of the filtrate, ethanol is added, and the solid precipitated is the unnatural sugar without protecting groups. In the above method for synthesizing unnatural sugars without protecting groups, all reactions are performed at room temperature, there is no chromatographic purification process, and large-scale synthesis (on the order of 10 grams) can be carried out. In the process of synthesizing unnatural sugars containing azide, only one equivalent of sodium azide is needed, which is small in amount and safe to operate. Based on this TMS protection strategy, the applicant synthesized GalNAz, GlcNAz, ManNAz and ManNAl lin large quantities.

In the above-mentioned process of synthesizing unnatural sugars without protecting groups, the per-trimethylsilane-protected unnatural sugar with orthogonal groups is an important intermediate. Starting from this intermediate, the 1,6-bis-acylated unnatural sugar can be obtained through two simple steps of reaction. For galactose-type and glucose-type per-TMS-protected unnatural sugars, the TMS at positions 1 and 6 of the per-TMS-protected unnatural sugars are exchanged into corresponding ester bonds by using pyridine as a solvent and adding four equivalents of carboxylic acid and a large excess of corresponding acid anhydride, and the TMS protecting groups at positions 3 and 4 are removed to obtain a 1,6-bis-acylated unnatural sugar; and for a mannose-type per-TMS-protected unnatural sugar, the TMS protecting groups at positions 1 and 6 of the sugar are selectively and simultaneously removed by adding two equivalents of ammonium acetate to a mixed solvent of dichloromethane and methanol, and positions 1 and 6 are protected with ester bonds in pyridine and the TMS protecting groups at positions 3 and 4 of the sugar are then removed to obtain a 1,6-bis-acylated mannose-type unnatural sugar derivative.

In the metabolic glycan labeling experiment, it is found that 1,6-bis-acylated sugar did not undergo S-glyco modification with proteins; and at the same time, 1,6-bis-acylated sugar has a higher metabolic labeling efficiency. In addition, compared with sugars without protecting groups, different ester bond protecting groups have different effects on improving metabolic labeling efficiency. For example, the efficiency improvement of acetyl is not significant compared with propionyl. Butyryl modification and propionyl modification can significantly improve the metabolic efficiency of unnatural sugars. Finally, 1,6-dipropionylated sugars can also be used for efficient metabolic labeling in vivo in mice.

In some embodiments of the present invention, when synthesizing unnatural sugars without protecting groups based on the per-TMS strategy, the orthogonal groups contained are not limited to azide and alkyne, and unnatural sugars without protecting groups containing other orthogonal groups can be synthesized using this strategy. Other orthogonal groups include, but are not limited to, terminal alkene groups, cyclopropene groups, trans-cyclooctene groups, and cyclooctyne groups. Therefore, the synthesis of these unprotected unnatural sugars is protected by this patent.

In some embodiments of the present invention, when synthesizing unnatural sugars without protecting groups based on the per-TMS strategy, the applicable substrates are not limited to galactosamine derivatives, glucosamine derivatives and mannosamine derivatives, and other amino-containing sugars can be used as substrates for this type of synthesis. Therefore, the synthesis of these unprotected unnatural sugars is protected by this patent.

In some embodiments of the present invention, when synthesizing partially acylated unnatural sugar based on the per-TMS strategy, the acyl groups used are not limited to acetyl, propionyl and butyryl, and other acyl groups can also be introduced using the method of the present application. Therefore, other acylation-protected partially acylated unnatural sugars are also protected by this patent.

In a first typical embodiment of the present application, a simple and efficient method is provided for synthesizing unnatural sugars without protecting groups, specifically GalNAz, GlcNAz, ManNAz and ManNAl, and synthesizing the partially acylated unnatural sugars mentioned above.

In a second typical embodiment of the present application, there is provided a metabolic glycan labeling reagent kit, comprising any one of the above-mentioned partially acylated unnatural sugars.

In a third typical embodiment of the present application, there is provided use of any of the above-mentioned partially acylated unnatural sugars or the above-mentioned reagent kit in metabolic glycan labeling. The use of the partially acylated unnatural sugar in the present application for metabolic labeling not only has a higher metabolic efficiency, but also avoid S-glyco modification with cysteine in proteins. In a preferred embodiment, the above use includes metabolic glycan labeling of any of the following cells: HeLa cells, MCF-7 cells, CHO cells and 3T3 cells. In a preferred embodiment, 1,6-Pr₂GalNAz and 1,6-Pr₂ManNAz are used for metabolic labelling in vivo in mice.

When synthesizing unnatural sugars containing azide with some hydroxyl groups protected in the prior art, sodium azide is required, which has the risk of explosion, especially when synthesizing in large quantities, such as on the order of 10 grams, the risk of explosion is even higher. The method for synthesizing 1,6-Pr₂GalNAz in the present invention is more efficient and safer than the synthesis methods reported in the literature. Compared with other reported 1,3-bis-acylated unnatural sugars, the 1,6-bis-acylated unnatural sugars of the present invention are a better choice based on the mechanism of the S-glycosylation modification side reaction on the protein thiol group and the stability of the partially acylated sugars. In addition, the synthesis of 1,6-bis-acylated sugars in the present invention is based on per-TMS-protected sugars, making the synthesis simpler and more efficient.

The beneficial effects of the present application are further illustrated below with reference to specific embodiments.

### Example 1: Synthesis of GalNAz, GlcNAz and ManNAz.

The synthesis of GalNAz, GlcNAz and ManNAz is shown in Synthesis Route 1:

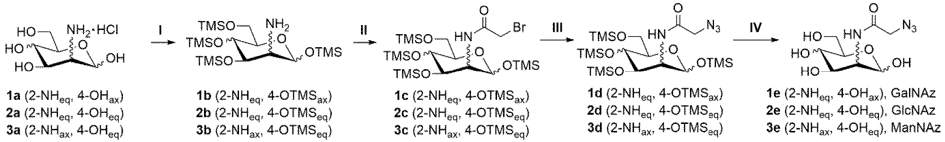

Wherein, the conditions of steps I , II, III, and IV are as follows:
GalN hydrochloride (Compound **1a,** 100 mmol, 21.5 g) was dispersed in 200 ml of anhydrous acetonitrile, and 40.3 g of HMDS (250 mmol) was added in batches. The reaction was carried out at room temperature for 3 hours. The mixture was filtered and the filtrate was concentrated in vacuo to obtain compound **1b,** which is an oily substance and can be used directly in the next step without purification.

27.6 g of bromoacetic acid (200 mmol) and 25.3 g of HOSU (220 mmol) were dissolved in 220 ml of dichloromethane, and the solution was cooled to -5°C. A dichloromethane solution (50 ml) containing 45.3 g (220 mmol) of DCC was added dropwise to the above solution over a period of 1 hour. After the addition was complete, the reaction system was heated to room temperature and allowed to react for 2 hours. The mixture was filtered and the filtrate was concentrated in vacuo to obtain a residue. The residue was treated with 200 ml of n-hexane and stirred thoroughly to obtain a mixture. The mixture was filtered, and the filter cake was washed with n-hexane to obtain a white solid. After vacuum drying, 36.0 g of crude product, 2,5-dioxopyrrolidin-1-yl2-bromoacetate **(4a)** were obtained. Compound **4a** was used directly for the next reaction without purification. All compound **1b** obtained in the previous step were dissolved in 200 ml of dichloromethane, cooled in an ice-water bath, and then 33.3 g of **4a** (140 mmol) were added. Stir at room temperature for 2 hours, concentrate under vacuum to constant weight, and add 200 ml of n-hexane and 20 ml of ethyl acetate and stir. After precipitation, filtration and spin drying of the filtrate, a yellow oily compound **1c** was obtained. **1c** was used directly for the next step without purification.

The compound **1c** obtained in the previous step was dissolved in 150 ml of DMF, 6.8 g (100 mmol) of sodium azide was added, and the mixture was stirred at room temperature overnight. The mixture was diluted with 200 ml of ethyl acetate, the organic phase was washed with water and the aqueous phase was extracted once with 100 ml of ethyl acetate. The organic phase was washed twice with saturated brine and dried over anhydrous sodium sulfate. After filtration and vacuum concentration of the filtrate, a yellow oily compound **1d** was obtained. **1d** was used directly for the next step without purification.

The compound **1d** was dissolved in 150 ml of methanol, 10 g of hydrogen ion exchange resin (Dowex) was added, and the mixture was stirred at room temperature for 2 hours, and TLC showed that the reaction was complete. After filtration and spin drying of the filtrate, 200 ml of ethanol was added and stirred, the mixture was filtered, and the filter cake was collected. After vacuum drying, 11.6 g of product **1e** was obtained, with a four-step yield of 44%, and the product was α- configuration.

The NMR detection data of compound **1e** (GalNAz) were as follows: ¹H NMR (500 MHz, DMSO-*d*₆) δ 7.78 (d, *J* = 8.7 Hz, 1H), 6.43 (dd, *J* = 4.4, 1.2 Hz, 1H), 4.95 (t, *J* = 3.9 Hz, 1H), 4.51 (t, *J* = 5.6 Hz, 1H), 4.47 (d, *J* = 4.3 Hz, 1H), 4.45 - 4.40 (m, 2H), 4.01 (ddd, *J* = 11.5, 8.7, 3.4 Hz, 1H), 3.85 (d, *J* = 15.4 Hz, 1H), 3.83 - 3.79 (m, 1H), 3.80 (d, *J* = 15.4 Hz, 1H), 3.76 - 3.70 (m, 1H), 3.68 - 3.58 (m, 1H), 3.58 - 3.50 (m, 1H), 3.43 (dd, *J* = 10.7, 6.2 Hz, 1H). ¹³C NMR (126 MHz, DMSO-*d*₆) δ 167.7, 90.9, 70.6, 68.3, 67.4, 60.7, 50.7, 50.6.

Compound **2e** (GlcNAz, 9.6 g) was synthesized from 21.5 g (100 mmol) of GlcN hydrochloride using the method for synthesizing GalNAz in Example 1 above. The four-step yield was 37%. The product was a mixture of end isomers, α/β, 5/1.

The NMR detection data of compound **2e** (GIcNAz) were as follows: ¹H NMR (α isomer, 500 MHz, Methanol-*d*₄) δ 5.11 (d, *J* = 3.5 Hz, 1H), 3.95 (d, *J* = 15.8 Hz, 1H), 3.92 (s, 1H), 3.87 (dd, *J =* 10.7, 3.7 Hz, 1H), 3.83 - 3.76 (m, 2H), 3.74 - 3.67 (m, 2H), 3.38 (t, *J* = 9.2 Hz, 1H). ¹³C NMR (126 MHz, Methanol-*d*₄) δ 170.9, 170.4, 96.8, 92.5, 78.1, 75.8, 73.2, 72.8, 72.4, 72.2, 62.9, 62.8, 59.0, 55.9, 53.2, 52.9.

Compound **3e** (ManNAz, 10.8 g) was synthesized from 21.5 g (100 mmol) of ManNAz hydrochloride using the method for synthesizing GalNAz in Example 1 above. The four-step yield was 41%. The product was a mixture of end isomers, α/β, 2/1.

The NMR detection data of compound **3e** (ManNAz) were as follows: ¹H NMR (500 MHz, Methanol-*d*₄) δ 5.03 (d, *J* = 1.6 Hz, 1H), 4.29 (dd, *J* = 4.7, 1.7 Hz, 1H), 4.02 (dd, *J* = 9.7, 4.7 Hz, 1H), 3.93 (s, 1H), 3.89 (d, *J* = 15.8 Hz, 1H), 3.86 - 3.74 (m, 3H), 3.55 (t, *J* = 9.6 Hz, 1H). ¹³C NMR (151 MHz, Methanol-*d*₄) δ 171.5, 170.6, 94.6, 94.5, 78.1, 74.1, 73.2, 70.2, 68.3, 68.0, 62.0, 61.9, 55.7, 55.0, 52.6, 52.4, 49.6.

### Example 2: Synthesis of ManNAl.

The synthesis of ManNAl is shown in Synthesis Route 2:

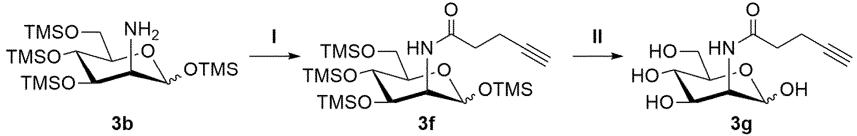

Wherein, the conditions of steps I and II are as follows:
2.16 g (22 mmol) of pentynoic acid and 2.78 g (24.2 mmol) of HOSU were dissolved in 50 ml of dichloromethane, and the solution was cooled to -5 °C. A dichloromethane solution (20 ml) containing 4.98 g (24.4 mmol) of DCC was added dropwise to the above solution over a period of 20 minutes. The reaction system was heated to room temperature and stirred for 2 hours until the reaction was complete. After filtration and concentration of the filtrate, a product **4b** (2,5-dioxopyrrolidin-1-yl pent-4-ynoate) was obtained. **4b** was used directly for the next step without purification.

A crude compound **3b** was synthesized from 18.7 mmol of ManN hydrochloride **(3a)** according to the method in Example 1. The crude compound **3b** was dissolved in 50 ml of dichloromethane, cooled to 0°C in an ice-water bath, and the compound **4b** obtained in the previous step was added dropwise to the solution. The mixture was stirred at room temperature for 2 hours. After completion of the reaction, the mixture was concentrated in vacuo to obtain a residue. Add 100 ml of n-hexane to the residue, stir and filter, and concentrate the filtrate in vacuo to obtain a yellow oily compound **3f,** which was used directly for the next step without purification.

The **3f** obtained in the previous step was dissolved in 50 ml of methanol, and 2 g of hydrogen ion exchange resin was added. Stir at room temperature for 2 hours and the reaction was complete. After filtration and vacuum concentration of the filtrate, a residue was obtained. The residue was purified by silica gel column to obtain 3.06 g of compound **3g** (ManNAI). The three-step yield was 64%. The product was a mixture of end isomers, α/β, 3/1.

The NMR detection data of compound **3g** were as follows: ¹H NMR (500 MHz, Methanol-*d*₄) δ 5.01 (d, *J* = 1.6 Hz, 1H), 4.29 (dd, *J* = 4.6, 1.7 Hz, 1H), 4.00 (dd, *J* = 9.7, 4.7 Hz, 1H), 3.87 - 3.74 (m, 3H), 3.58 (t, *J* = 9.7 Hz, 1H), 3.35 (s, 1H), 2.59 - 2.42 (m, 4H), 2.26 (t, *J* = 2.3 Hz, 1H). ¹³C NMR (126 MHz, Methanol-*d*₄) δ 175.5, 174.5, 94.8, 94.7, 83.8, 83.5, 78.0, 74.3, 73.2, 70.4, 70.0, 69.9, 68.3, 67.9, 62.0, 61.9, 55.6, 54.9, 35.9, 35.72, 35.67, 15.42, 15.40.

### Example 3: Synthesis of 1,6-Ac₂GalNAz, 1,6-Pr₂GalNAz, 1,6-Bu₂GalNAz, 6-Ac₂GlcNAz, 1,6-Pr₂GlcNAz and 1,6-Bu₂GlcNAz.

The synthesis of 1,6-Ac₂GalNAz, 1,6-Pr₂GalNAz, 1,6-Bu₂GalNAz, 6-Ac₂GlcNAz, 1,6-Pr₂GlcNAz, and 1,6-Bu₂GlcNAz is shown in Synthesis Route 3:

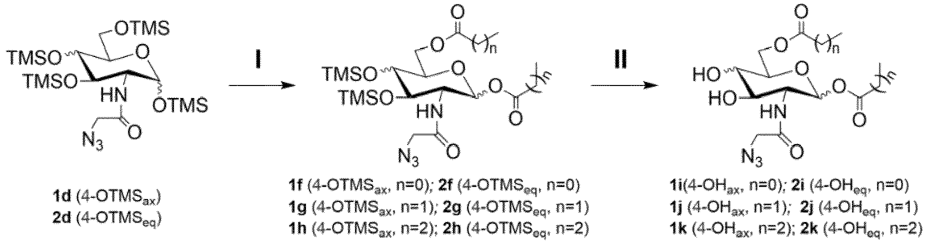

Wherein, the conditions of steps I and II are as follows:
Step I, general method for the synthesis of **1f, 1g, 1h, 2f, 2g** and **2h:** 0.5 mmol of **1d** or **2d** was dissolved in 1 ml of pyridine, and 0.75 ml of anhydride and 2.0 mmol of the corresponding carboxylic acid were added under nitrogen protection. The reaction was carried out at room temperature for 20 hours, the reaction solution was diluted with ethyl acetate, and the organic phase was washed with 1 mol/L dilute hydrochloric acid, saturated sodium bicarbonate solution and saturated brine in sequence. The organic phase was dried over anhydrous sodium sulfate, filtered after drying, the filtrate was concentrated in vacuo, and the residue was purified by silica gel column to obtain the corresponding product.

**1d. 1d** is an intermediate in the synthesis of **1e** from **1a** and can be purified by flash chromatography using a silica gel column with petroleum ether and ethyl acetate as eluents. 4.1 g of **1d** was synthesized from 20 mmol of **1a** with a three-step yield of 37%. The product was configuration.

The NMR detection data of compound **1d** were as follows: ¹H NMR (500 MHz, Chloroform-*d*) δ 6.29 (d, *J* = 8.0 Hz, 1H), 5.16 - 5.10 (m, 1H), 4.10 (dd, *J* = 10.4, 2.8 Hz, 1H), 3.92 (s, 2H), 3.87 (d, *J* = 2.8 Hz, 1H), 3.68 - 3.62 (m, 1H), 3.62 - 3.55 (m, 1H), 3.55 - 3.49 (m, 1H), 3.47 - 3.43 (m, 1H), 0.13 (s, 9H), 0.12 (s, 9H), 0.12 (s, 9H), 0.11 (s, 9H). ¹³C NMR (126 MHz, Chloroform-d) δ 166.70, 94.63, 75.17, 71.24, 70.63, 61.04, 57.42, 53.18, 0.76, 0.44, 0.30, -0.40.

**2d.** The synthesis and purification of **2d** was similar to those of **1d.** 5.91 g of **2d** was synthesized from 20 mmol of **2a** with a three-step yield of 54%. The product was configuration.

The NMR detection data of compound **2d** were as follows: ¹H NMR (400 MHz, Chloroform-*d*) δ 6.36 (d, *J* = 10.1 Hz, 1H), 5.01 (d, *J* = 3.5 Hz, 1H), 4.07 - 3.94 (m, 3H), 3.76 - 3.64 (m, 3H), 3.63 - 3.56 (m, 2H), 0.17 (s, 9H), 0.15 (s, 9H), 0.12 (s, 9H), 0.09 (s, 9H). ¹³C NMR (126 MHz, Chloroform-*d*) δ 166.24, 92.59, 74.04, 72.71, 72.07, 61.75, 54.53, 53.11, 1.05, 0.90, -0.13, -0.21.

**1f. 1f** was synthesized according to the general method in Example 3 above. The yield was 86%. The product was an end isomer, α/β, 1/1.56.

The NMR detection data of **1f** were as follows: ¹H NMR (β isomer, 500 MHz, Chloroform-d) δ 6.20 (d, *J* = 8.8 Hz, 1H), 5.90 (d, *J* = 8.6 Hz, 1H), 4.18 (d, *J* = 2.0 Hz, 1H), 4.17 - 4.13 (m, 1H), 4.04 (dt, *J* = 10.5, 8.7 Hz, 1H), 3.94 (dd, *J* = 11.0, 2.7 Hz, 3H), 3.83 (d, *J* = 2.7 Hz, 1H), 3.78 (td, *J* = 6.3, 0.9 Hz, 1H), 2.10 (s, 3H), 2.07 (s, 3H), 0.14 (s, 9H), 0.14 (s, 9H). ¹H NMR (α end isomer, 500 MHz, Chloroform-d) δ 6.16 (d, *J* = 3.7 Hz, 1H), 6.03 (d, *J* = 9.4 Hz, 1H), 4.63 (td, *J* = 10.0, 3.6 Hz, 1H), 4.13 (d, *J* = 6.4 Hz, 2H), 4.05 (d, *J* = 16.8 Hz, 1H), 3.99 (d, *J* = 16.6 Hz, 1H), 3.98 (t, *J* = 6.3 Hz, 1H), 3.89 (d, *J* = 1.8 Hz, 1H), 3.81 (dd, *J* = 10.6, 2.6 Hz, 1H), 2.15 (s, 3H), 2.07 (s, 3H), 0.18 (s, 9H), 0.15 (s, 9H). ¹³C NMR (α/β, 126 MHz, Chloroform-d) δ 170.73, 170.69, 169.7, 169.2, 166.8, 166.3, 92.3, 92.1, 73.55, 71.9, 71.2, 71.1, 70.8, 69.6, 63.2, 63.2, 52.9, 52.8, 52.8, 48.3, 21.1, 21.0, 20.9, 0.63, 0.55, 0.4, 0.36.

**1g. 1g** was synthesized according to the general method in Example 3 above. The yield was 89%. The product was an end isomer, α/β, 1.25/1.

The NMR detection data of compound **1g** were as follows: ¹H NMR (a mixture of α and β end isomer, 500 MHz, Chloroform-d) δ 6.21 (d, *J* = 8.9 Hz, 1H), 6.18 (d, *J* = 3.6 Hz, 1H), 6.04 (d, *J* = 9.5 Hz, 1H), 5.90 (d, *J* = 8.6 Hz, 1H), 4.64 (td, *J* = 10.1, 3.6 Hz, 1H), 4.19 (d, *J* = 6.3 Hz, 2H), 4.15 (d, *J* = 6.5 Hz, 2H), 4.11 - 4.02 (m, 2H), 4.02 - 3.96 (m, 2H), 3.94 (d, *J* = 5.4 Hz, 2H), 3.91 - 3.88 (m, 1H), 3.85 - 3.80 (m, 2H), 3.80 - 3.76 (m, 1H), 2.46 - 2.30 (m, 8H), 1.23 - 1.09 (m, 12H), 0.18 (s, 9H), 0.15 (s, 19H), 0.14 (s, 8H). ¹³C NMR (a mixture of α and β end isomer, 126 MHz, Chloroform-d) δ 174.2, 174.2, 173.2, 172.6, 166.8, 166.2, 92.3, 91.9, 73.6, 72.0, 71.2, 71.1, 70.8, 69.7, 63.0, 62.9, 52.9, 52.8, 52.8, 48.3, 27.8, 27.6, 27.55, 27.52, 9.2, 9.17, 9.14, 8.9, 0.63, 0.56, 0.4, 0.3.

**1h. 1h** was synthesized according to the general method in Example 3 above. The yield was 70%. The product was a mixture of end isomers, α/β, 2.55/1.

The NMR detection data of compound **1h** were as follows: ¹H NMR (500 MHz, Chloroform-d) δ 6.18 (d, *J* = 3.7 Hz, 1H), 6.02 (d, *J* = 9.5 Hz, 1H), 4.63 (td, *J* = 10.0, 3.6 Hz, 1H), 4.03 (d, *J* = 16.9 Hz, 1H), 4.00 (s, 1H), 3.98 - 3.95 (m, 1H), 3.92 (d, *J* = 4.2 Hz, 1H), 3.90 (d, *J* = 2.6 Hz, 1H), 3.83 (dd, *J* = 4.9, 2.6 Hz, 1H), 2.36 (t, *J* = 7.3 Hz, 2H), 2.28 (t, *J* = 7.3 Hz, 2H), 1.73 - 1.66 (m, 2H), 1.66 - 1.59 (m, 2H), 0.99 (t, *J* = 7.3 Hz, 3H), 0.94 (t, *J* = 7.4 Hz, 3H), 0.17 (s, 9H), 0.14 (s, 9H). ¹³C NMR (126 MHz, Chloroform-d) δ 173.1, 173.0, 172.0, 171.4, 166.5, 166.0, 91.8, 91.5, 73.2, 71.8, 71.0, 70.9, 70.5, 69.4, 62.8, 62.5, 52.6, 52.4, 52.4, 48.0, 36.0, 35.8, 35.8, 35.4, 18.2, 18.2, 18.0, 17.9, 13.40, 13.37, 13.3, 13.2, 0.33, 0.26, 0.1, 0.01.

**2f. 2f** was synthesized according to the general method in Example 3 above. The yield was 62%. α isomer.

The NMR detection data of compound **2f** were as follows: ¹H NMR (500 MHz, Methanol-*d*₄) δ 5.97 (d, *J* = 3.9 Hz, 1H), 4.37 (dd, *J* = 12.1, 2.3 Hz, 1H), 4.18 (dd, *J* = 10.1, 3.9 Hz, 1H), 4.07 (dd, *J* = 12.1, 4.3 Hz, 1H), 3.89 - 3.84 (m, 3H), 3.82 (dd, *J* = 10.1, 8.1 Hz, 1H), 3.69 (dd, *J* = 9.7, 8.1 Hz, 1H), 2.16 (s, 3H), 2.08 (s, 3H), 0.19 (s, 9H), 0.17 (s, 9H). ¹³C NMR (126 MHz, Methanol-*d*₄) δ 172.6, 171.4, 170.8, 92.4, 75.1, 74.0, 73.4, 64.4, 54.5, 53.0, 21.1, 21.0, 1.4, 1.2.

**2g. 2g** was synthesized according to the general method in Example 3 above. The yield was 62%. α isomer.

The NMR detection data of compound **2g** were as follows: ¹H NMR (500 MHz, Chloroform-*d*) δ 6.35 (d, *J* = 9.9 Hz, 1H), 6.06 (d, *J* = 3.5 Hz, 1H), 4.39 (dd, *J* = 12.1, 2.7 Hz, 1H), 4.28 (td, *J* = 9.6, 3.5 Hz, 1H), 4.10 (dd, *J* = 12.1, 4.8 Hz, 1H), 4.06 (d, *J* = 16.8 Hz, 1H), 4.00 (d, *J* = 16.8 Hz, 1H), 3.89 - 3.80 (m, 1H), 3.75 (dd, *J* = 9.3, 7.6 Hz, 1H), 3.68 (dd, *J* = 8.5, 7.6 Hz, 1H), 2.42 (qd, *J* = 7.5, 0.9 Hz, 2H), 2.36 (qd, *J* = 7.6, 2.9 Hz, 2H), 1.19 (t, *J* = 7.5 Hz, 3H), 1.14 (t, *J* = 7.5 Hz, 3H), 0.16 (s, 9H), 0.16 (s, 9H). ¹³C NMR (151 MHz, Chloroform-d) δ 173.8, 172.2, 166.0, 90.4, 73.2, 72.9, 71.3, 62.2, 52.4, 51.8, 27.4, 27.2, 8.82, 8.78.

**2h. 2h** was synthesized according to the general method in Example 3 above. The yield was 44%. α isomer.

The NMR detection data of compound **2h** were as follows: ¹H NMR (500 MHz, Chloroform-*d*) δ 6.32 (d, *J* = 9.9 Hz, 1H), 6.07 (d, *J* = 3.5 Hz, 1H), 4.40 (dd, *J* = 12.1, 2.7 Hz, 1H), 4.28 (td, *J* = 9.7, 3.6 Hz, 1H), 4.08 (dd, *J* = 12.1, 4.9 Hz, 1H), 4.06 (d, *J* = 16.8 Hz, 1H), 4.00 (d, *J* = 16.8 Hz, 1H), 3.83 (ddd, *J* = 7.8, 4.8, 2.6 Hz, 1H), 3.75 (dd, *J* = 9.4, 7.7 Hz, 1H), 3.67 (dd, *J* = 8.7, 7.7 Hz, 1H), 2.37 (t, *J* = 7.3 Hz, 2H), 2.31 (td, *J* = 7.4, 3.7 Hz, 2H), 1.72 - 1.63 (m, 4H), 0.99 (t, *J* = 7.4 Hz, 3H), 0.95 (t, *J* = 7.4 Hz, 3H), 0.17 (s, 9H), 0.16 (s, 9H). ¹³C NMR (126 MHz, Chloroform-d) δ 172.4, 170.8, 165.5, 89.8, 72.6, 70.8, 61.6, 51.8, 51.3, 35.3, 35.2, 17.6, 17.5, 12.8, 12.7, 1.4, 1.2.

General method for the synthesis of **1i, 1j, 1k, 2i, 2j** and **2k:** Add an appropriate amount of hydrogen ion exchange resin to a methanol solution of **1f, 1g, 1h, 2f, 2g** or **2h** and stir at room temperature. After TLC showed the reaction is complete, filter and concentrate the filtrate in vacuo, and purify the residue by silica gel column to obtain the corresponding product.

**1i. 1i** was synthesized according to the general method in Example 3 above. The yield was 52%, α/β, 2/1.

The NMR and mass spectrometry detection data of compound **1i** were as follows: ¹H NMR (α isomer, 500 MHz, Methanol-*d*₄) δ 6.15 (d, *J* = 3.7 Hz, 1H), 4.42 (dd, *J* = 11.1, 3.7 Hz, 1H), 4.22 (d, *J* = 3.3 Hz, 1H), 4.21 (d, *J* = 1.5 Hz, 1H), 4.11 (ddd, *J* = 6.8, 5.1, 1.3 Hz, 1H), 3.94 (dd, *J* = 3.2, 1.3 Hz, 1H), 3.93 - 3.81 (m, 4H), 2.12 (s, 3H), 2.04 (s, 3H). ¹³C NMR (a mixture of α and β isomer, 126 MHz, Methanol-*d*₄) δ 171.2, 169.9, 169.7, 169.6, 169.5, 92.8, 91.0, 73.6, 70.78, 70.77, 68.4, 69.0, 67.3, 63.4, 63.3, 51.8, 51.6, 51.3, 49.1, 19.4, 19.3. HRMS (ESI) theoretical molecular weight: C₁₂H₁₉N₄O₈ [M+H]⁺ 347.12029, detected molecular weight: 347.11959.

**1j. 1j** was synthesized according to the general method in Example 3 above. The yield was 83%, α/β, 1.45/1.

The NMR and mass spectrometry detection data of compound **1j** were as follows: ¹H NMR (α isomer, 500 MHz, Methanol-*d*₄) δ 6.16 (d, *J* = 3.7 Hz, 1H), 4.41 (dd, *J* = 11.1, 3.7 Hz, 1H), 4.10 (ddd, *J* = 6.8, 5.1, 1.3 Hz, 1H), 3.94 (dd, *J* = 3.3, 1.3 Hz, 1H), 3.90 (d, *J* = 15.8 Hz, 1H), 3.88 (dd, *J* = 11.1, 3.2 Hz, 1H), 3.83 (d, *J* = 15.8 Hz, 1H), 2.42 (qd, *J* = 7.6, 1.9 Hz, 2H), 2.33 (q, *J* = 7.5 Hz, 2H), 1.14 (t, *J* = 7.5 Hz, 3H), 1.10 (t, *J* = 7.6 Hz, 3H). ¹³C NMR (126 MHz, Methanol-*d*₄) δ 175.71, 175.68, 174.4, 174.2, 170.7, 170.6, 94.0, 92.0, 74.8, 72.0, 71.9, 69.6, 69.1, 68.6, 64.4, 64.2, 53.0, 52.8, 52.5, 50.4, 28.0, 28.0, 27.97, 27.94, 9.2, 9.14, 9.06, 8.9. HRMS (ESI) theoretical molecular weight: C₁₄H₂₃N₄O₃ [M+H]⁺ 375.15159, detected molecular weight: 375.15076.

**1k. 1k** was synthesized according to the general method in Example 3 above. The yield was 69%, α/β, 2.67/1.

The NMR and mass spectrometry detection data of compound **1k** were as follows: ¹H NMR (α isomer, 500 MHz, Methanol-*d*₄) δ 6.17 (d, *J* = 3.7 Hz, 1H), 4.41 (dd, *J* = 11.1, 3.7 Hz, 1H), 4.34 - 4.19 (m, 3H), 4.09 (ddd, *J* = 7.4, 4.7, 1.3 Hz, 1H), 3.94 (dd, *J* = 3.2, 1.3 Hz, 1H), 3.92 - 3.80 (m, 5H), 2.39 (t, *J* = 7.3 Hz, 2H), 2.35 - 2.27 (m, 4H), 1.72 - 1.57 (m, 6H), 1.00 - 0.91 (m, 9H). ¹³C NMR (126 MHz, Methanol-*d*₄) δ 173.7, 173.6, 172.3, 172.2, 169.5, 169.4, 92.7, 90.7, 73.6, 70.90, 70.86, 68.5, 68.0, 67.4, 63.4, 63.0, 51.8, 51.6, 51.3, 49.2, 35.5, 35.42, 35.41, 18.00, 17.96, 17.8, 12.5, 12.5, 12.4. HRMS (ESI) theoretical molecular weight: C₁₆H₂₇N₄O₈ [M+H]⁺ 403.18289, detected molecular weight: 403.18234.

**2i. 2i** was synthesized according to the general method in Example 3 above. The yield was 69%. α isomer.

The NMR and mass spectrometry detection data of compound **2i** were as follows: ¹H NMR (500 MHz, Methanol-*d*₄) δ 6.10 (d, *J* = 3.7 Hz, 1H), 4.33 (dd, *J* = 12.1, 2.3 Hz, 1H), 4.23 (dd, *J* = 12.1, 5.2 Hz, 1H), 4.04 (dd, *J* = 10.8, 3.6 Hz, 1H), 3.90 (d, *J* = 15.8 Hz, 1H), 3.87 - 3.80 (m, 2H), 3.73 (dd, *J* = 10.8, 8.8 Hz, 1H), 3.45 (dd, *J* = 10.2, 8.8 Hz, 1H), 2.13 (s, 3H), 2.06 (s, 3H). ¹³C NMR (126 MHz, Methanol-*d*₄) δ 172.7, 171.1, 170.8, 91.9, 73.5, 72.0, 71.8, 64.4, 54.4, 52.6, 20.7, 20.6. HRMS (ESI) theoretical molecular weight: C₁₂H₁₉N₄O₈ [M+H]⁺ 347.12029, detected molecular weight: 347.11922.

**2j. 2j** was synthesized according to the general method in Example 3 above. The yield was 79%. α isomer.

The NMR and mass spectrometry detection data of compound **2j** were as follows: ¹H NMR (500 MHz, Methanol-*d*₄) δ 6.12 (d, *J* = 3.6 Hz, 1H), 4.35 (dd, *J* = 12.0, 2.2 Hz, 1H, C6-Ha), 4.23 (dd, *J* = 12.0, 5.4 Hz, 1H, C6-Hb), 4.04 (dd, *J* = 10.8, 3.7 Hz, 1H), 3.90 (d, *J* = 15.9 Hz, 1H, CH2a), 3.87 - 3.80 (m, 2H, C5-H), 3.73 (dd, *J* = 10.8, 8.8 Hz, 1H), 3.45 (dd, *J* = 10.1, 8.8 Hz, 1H), 2.45 (qd, *J* = 7.5, 1.1 Hz, 2H), 2.36 (q, *J* = 7.6 Hz, 2H), 1.15 (t, *J* = 6.8 Hz, 3H), 1.12 (t, 3H). ¹³C NMR (126 MHz, Methanol-*d*₄) δ 175.8, 174.2, 170.6, 91.6, 73.3, 71.9, 71.6, 64.1, 54.3, 52.4, 28.0, 9.2, 9.0. HRMS (ESI) theoretical molecular weight: C₁₄H₂₃N₄O₈ [M+H]⁺ 375.15159, detected molecular weight: 375.15102.

**2k. 2k** was synthesized according to the general method in Example 3 above. The yield was 78%. α isomer.

The NMR and mass spectrometry detection data of compound **2k** were as follows: ¹H NMR (500 MHz, Methanol-*d*₄) δ 6.12 (d, *J* = 3.6 Hz, 1H), 4.37 (dd, *J* = 11.9, 2.2 Hz, 1H), 4.21 (dd, *J* = 12.0, 5.7 Hz, 1H), 4.03 (dd, *J* = 10.8, 3.7 Hz, 1H), 3.90 (d, *J* = 15.8 Hz, 1H), 3.85 - 3.80 (m, 2H), 3.73 (dd, *J* = 10.8, 8.8 Hz, 1H), 3.43 (dd, *J* = 10.1, 8.8 Hz, 1H), 2.40 (td, *J* = 7.2, 1.2 Hz, 2H), 2.32 (t, *J* = 7.3 Hz, 2H), 1.72 - 1.66 (m, 2H), 1.67 - 0.96 (m, 8H), 0.98 (t, *J* = 7.4 Hz, 3H), 0.95 (t, *J* = 7.4 Hz, 3H). ¹³C NMR (126 MHz, Methanol-*d*₄) δ 174.9, 173.3, 170.5, 91.4, 73.4, 71.9, 71.7, 64.1, 54.3, 52.4, 36.6, 36.6, 19.2, 19.1, 13.72, 13.68. HRMS (ESI) theoretical molecular weight: C₁₆H₂₇N₄O₈ [M+H]⁺ 403.18289, detected molecular weight: 403.18225.

### Example 4: Synthesis of 1,6-Ac₂ManNAz, 1,6-Pr₂ManNAz, 1,6-Bu₂ManNAz, 1,6-Pr₂ManNAI and 1,6-Pr₂ManNProc.

The synthesis of 1,6-Ac₂ManNAz, 1,6-Pr₂ManNAz, 1,6-Bu₂ManNAz, 1,6-Pr₂ManNAI and 1,6-Pr₂ManNProc is shown in Synthesis Route 4:

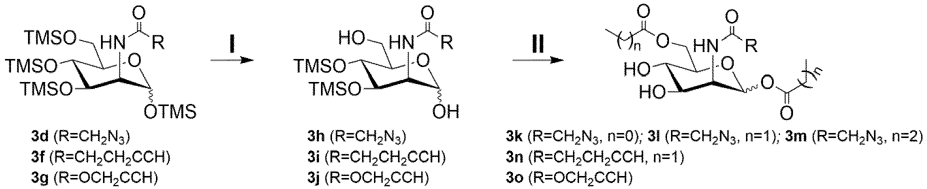

Wherein, the conditions of steps I and II are as follows:
**3d.** The synthesis and purification of **3d** was similar to those of **1d.** 4.1 g of **3d** was synthesized from 20 mmol of **3a** with a three-step yield of 37%. α isomer.

The NMR detection data of compound **3d** was as follows: ¹H NMR (500 MHz, Chloroform-*d*) δ 6.43 (d, *J* = 7.9 Hz, 1H), 5.10 (d, *J* = 1.5 Hz, 1H), 4.15 - 4.06 (m, 2H), 4.00 (d, *J* = 16.5 Hz, 1H), 3.95 (d, *J* = 16.3 Hz, 1H), 3.77 (dd, *J* = 11.3, 3.4 Hz, 1H), 3.72 - 3.60 (m, 3H), 0.16 (s, 9H), 0.16 (s, 9H), 0.15 (s, 9H), 0.12 (s, 9H). ¹³C NMR (126 MHz, Chloroform-d) δ 166.77, 93.39, 72.77, 70.10, 68.85, 61.65, 55.60, 53.00, 0.74, 0.37, -0.04, -0.25.

**3f.** 3.95g (8.44 mmol, 1.0 eq) of **3b** was dissolved in 50 ml of anhydrous dichloromethane, and 1.65 g (8.44 mmol, 1.0 eq) of 2,5-dioxopyrrolidin-1-yl pent-4-ynoate **(4b)** was added. Stir at room temperature for 2 hours. TLC showed that the reaction is complete. Spin dry the solvent to obtain a residue. Purify the residue by silica gel column to obtain 4.40 g of product **3f.** The yield was 95%. α isomer.

The NMR test results of compound **3f** were as follows: ¹H NMR (500 MHz, Chloroform-*d*) δ 5.78 (d, *J* = 7.1 Hz, 1H), 5.14 (d, *J* = 1.4 Hz, 1H), 4.11 - 4.05 (m, 2H), 3.73 (dd, *J* = 11.4, 4.2 Hz, 1H), 3.69 (dd, *J* = 11.4, 2.1 Hz, 1H), 3.65 (ddd, *J* = 9.6, 4.2, 2.1 Hz, 1H), 3.55 (t, *J* = 9.0 Hz, 1H), 2.55 - 2.48 (m, 2H), 2.46 - 2.40 (m, 2H), 2.01 (t, *J* = 2.6 Hz, 1H), 0.15 (s, 18H), 0.13 (s, 9H), 0.11 (s, 9H). ¹³C NMR (126 MHz, Chloroform-d) δ 171.14, 93.38, 83.12, 72.51, 70.08, 69.54, 69.06, 61.87, 55.71, 35.55, 14.84.

**3g.** 3.95 g (8.44 mmol, 1.0 eq) of **3b** was dissolved in 50 ml of a mixed solvent of anhydrous dichloromethane and pyridine (volume ratio, 1/1), cooled to 0°C in an ice-water bath, and 1.0 g (8.44 mmol, 1.0 eq) of prop-2-yn-1-yl carbonochloridate **(4c)** was added. The reaction system was heated to room temperature over 2 hours and stirred overnight. TLC showed that the reaction was complete. Concentrate in vacuo to obtain a residue and purify the residue by silica gel column to obtain 4.59 g of **3g** product. The yield was 99%. α isomer.

The NMR detection data of compound **3g** was as follows: ¹H NMR (500 MHz, Chloroform-*d*) δ 5.17 (d, *J* = 1.8 Hz, 1H), 4.99 (d, *J* = 7.5 Hz, 1H), 4.68 (d, *J* = 2.6 Hz, 2H), 4.05 (dd, *J* = 9.1, 4.7 Hz, 1H), 3.79 (ddd, *J* = 7.0, 4.7, 1.7 Hz, 1H), 3.69 (d, *J* = 3.3 Hz, 2H), 3.64 (dt, *J* = 9.3, 3.2 Hz, 1H), 3.49 (t, *J* = 9.2 Hz, 1H), 2.48 (t, *J* = 2.5 Hz, 1H), 0.15 (s, 9H), 0.14 (d, *J* = 2.0 Hz, 18H), 0.10 (s, 9H). ¹³C NMR (126 MHz, Chloroform*-d*) δ 155.51, 93.46, 78.27, 74.68, 72.70, 70.19, 68.94, 61.88, 57.33, 52.58, 0.76, 0.28, - 0.10, -0.27.

**3h.** 16.80 g of **3d** (30.49 mmol, 1.0 eq) was dissolved in a mixed solvent of 125 ml of dichloromethane and 125 ml of methanol, and 4.70 g (60.98 mmol, 2.0 eq) of ammonium acetate was added. Stir at room temperature for 16 hours, concentrate in vacuo, treat with 100 ml of ethyl acetate, filter, concentrate the filtrate and purify by a silica gel column to obtain 7.69 g of **3h.** The yield was 62%. The product was an end isomer, α/β, 1.0/0.3.

The NMR detection data of compound **3h** were as follows: ¹H NMR (500 MHz, DMSO-*d*₆) δ 7.75 (d, *J* = 8.8 Hz, 1H), 7.42 (d, *J* = 10.0 Hz, 0.3H), 6.66 (d, *J* = 4.5 Hz, 1H), 6.60 (d, *J* = 6.9 Hz, 0.3H), 4.85 (dd, *J* = 4.5, 1.3 Hz, 1H), 4.79 (dd, *J* = 6.9, 1.3 Hz, 0.3H), 4.42 - 4.37 (m, 1.3H), 4.20 (dd, *J* = 10.0, 3.1 Hz, 0.3H), 4.09 - 4.00 (ddd, *J* =8.8, 4.7, 1.5 Hz, 1H), 3.94 - 3.81 (m, 3.6H), 3.69 - 3.54 (m, 3.6H), 3.54 - 3.42 (m, 1.6H), 3.18 - 3.13 (m, 0.3H). ¹³C NMR (126 MHz, DMSO-*d*₆) δ 167.92, 167.66, 92.90, 92.49, 76.83, 73.29, 72.68, 70.17, 68.63, 68.51, 60.79, 60.71, 54.49, 54.16, 50.90, 50.64, 40.02, 39.86, 39.69, 39.52, 39.35, 39.19, 39.02, 0.81, 0.77, 0.19, 0.16. HRMS (ESI) theoretical molecular weight: C₁₄H₃₁N₄O₆Si₂ [M+H]⁺ 407.59400, detected molecular weight: 407.59736.

**3i. 3i** was synthesized from **3f** by referring to the synthesis of **3h** in Example 4. The yield was 67%. The product was an end isomer, α/β, 4/1.

The NMR detection data of compound **3i** were as follows: ¹H NMR (500 MHz, Methanol-*d*₄) δ 4.96 (d, *J* = 1.6 Hz, 1H), 4.27 (dd, *J* = 4.6, 1.7 Hz, 1H), 4.03 (dd, *J* = 8.2, 4.6 Hz, 1H), 3.81 - 3.74 (m, 4H), 3.73 - 3.69 (m, 1H), 2.54 - 2.42 (m, 5H), 2.26 - 2.22 (m, 1H), 0.18 - 0.14 (m, 22H). ¹³C NMR (126 MHz, Methanol-*d*₄) δ 175.19, 95.96, 84.61, 74.69, 72.91, 71.31, 70.90, 62.97, 56.60, 37.03, 16.97, 1.93, 1.42.

**3j. 3j** was synthesized from **3g** by referring to the synthesis of **3h** in Example 4. The yield was 63%. The product was an end isomer, α/β, 1.0/0.11.

The NMR detection data of compound **3j** were as follows: ¹H NMR (500 MHz, Chloroform-*d*) δ 5.34 (s, 1H), 5.16 (d, *J* = 6.4 Hz, 1H), 4.69 (d, *J* = 2.5 Hz, 2H), 4.06 (dt, *J* = 10.3, 5.1 Hz, 1H), 3.92 (ddd, *J* = 6.6, 4.7, 1.7 Hz, 1H), 3.85 (ddd, *J* = 9.4, 4.8, 2.5 Hz, 1H), 3.77 (dd, *J* = 11.8, 2.6 Hz, 1H), 3.69 (dd, *J* = 11.7, 5.4 Hz, 1H), 3.58 - 3.49 (m, 1H), 3.37 - 3.26 (m, 1H), 2.49 (t, *J* = 2.4 Hz, 1H), 0.16 (s, 9H), 0.15 (s, 9H). ¹³C NMR (126 MHz, Chloroform-*d*) δ 155.72, 93.12, 77.72, 75.33, 74.96, 70.00, 68.80, 61.85, 55.95, 53.42, 0.71, 0.23.

**3k.** 550 mg (1.35 mmol, 1.0 eq) of **3h** was dissolved in 10 ml of pyridine, cooled to 0°C in an ice-water bath, 552 mg (5.41 mmol, 4.0 eq) of acetic anhydride was added under nitrogen protection, the reaction system was gradually heated to room temperature and allowed to react for 18 hours, and 218 µl of methanol was added to quench the reaction. After stirring at room temperature for 1 hour, concentrate in vacuo to constant weight. The residue was dissolved in 50 ml of methanol, 5 g of hydrogen ion exchange resin was added, and stirred at room temperature for 2 hours. TLC showed that the reaction is complete. Filter, concentrate the filtrate in vacuo and purify the residue by silica gel column to obtain 331 mg of compound **3k.** The two-step yield was 68%. α/β, 1.0/0.28.

The NMR detection data of compound **3k** were as follows: ¹H NMR (500 MHz, Methanol-*d*₄, α isomer) δ 5.95 (d, *J* = 1.8 Hz, 1H), 4.33 (dd, *J* = 11.9, 2.3 Hz, 1H), 4.29 (dd, *J* = 4.8, 1.9 Hz, 1H), 4.25 (dd, *J* = 12.0, 6.5 Hz, 1H), 3.97 (dd, *J* = 9.6, 4.7 Hz, 1H), 3.95 (s, 1H), 3.94 (s, 1H), 3.81 (ddd, *J* = 10.1, 6.4, 2.4 Hz, 1H), 3.58 (t, *J* = 9.7 Hz, 1H), 2.12 (s, 3H), 2.05 (s, 3H). ¹³C NMR (151 MHz, Methanol-*d*₄, α isomer) δ 172.6, 170.8, 170.1, 101.1, 93.2, 73.7, 69.8, 68.2, 64.6, 53.3, 52.3, 20.50, 20.47. ¹H NMR (500 MHz, Methanol-d₄, β isomer) δ 5.79 (d, *J* = 1.8 Hz, 1H), 4.58 (dd, *J* = 4.6, 1.8 Hz, 1H), 4.42 (dd, *J* = 12.0, 2.2 Hz, 1H), 4.29 (dd, *J* = 11.9, 7.0 Hz, 1H), 3.99 (s, 1H), 3.99 (s, 1H), 3.80 (dd, *J* = 9.3, 4.6 Hz, 1H), 3.61 (ddd, *J* = 9.3, 7.0, 2.3 Hz, 1H), 3.48 (t, *J* = 9.5 Hz, 1H), 2.08 (s, 3H), 2.06 (s, 3H). ¹³C NMR (126 MHz, Methanol-d₄, β isomer) δ 170.9, 169.4, 168.5, 91.2, 75.0, 71.0, 66.7, 63.0, 51.4, 50.8, 18.84, 18.77. HRMS (ESI) theoretical molecular weight: C₁₂H₁₉N₄O₈ [M+H]⁺ 347.12029, detected molecular weight: 347.11914.

**3l. 3l** was obtained by the synthetic route of **3k** in Example 4, with 3h and propionic anhydride as raw materials. The two-step yield was 81%. α isomer.

The NMR detection data of compound **3l** were as follows: ¹H NMR (600 MHz, D₂O) δ 5.96 (s, 1H), 4.47 (dd, *J* = 4.7, 1.3 Hz, 1H), 4.42 (dd, *J* = 12.2, 2.0 Hz, 1H), 4.30 (dd, *J* = 12.2, 6.2 Hz, 1H), 4.15 (dd, *J* = 9.8, 4.8 Hz, 1H), 4.11 (s, 2H), 3.98 - 3.94 (m, 1H), 3.70 (t, *J* = 9.9 Hz, 1H), 2.51 (qd, *J* = 7.5, 1.9 Hz, 2H), 2.44 (q, *J* = 7.5 Hz, 2H), 1.14 (t, *J* = 7.6 Hz, 3H), 1.11 (t, *J* = 7.6 Hz, 3H). ¹³C NMR (151 MHz, D₂O) δ 177.3, 175.2, 171.1, 92.0, 72.1, 68.5, 66.6, 63.3, 51.71, 51.58, 27.21, 8.32, 8.15. HRMS (ESI) theoretical molecular weight: C₁₄H₂₃N₄O₈ [M+H]⁺ 375.15159, detected molecular weight: 375.15095.

**3m. 3m** was prepared by the synthetic route of **3k** in Example 4, with **3h** and butyric anhydride as raw materials. The two-step yield was 79%. α/β, 1.35/1.

The NMR detection data of **3m** were as follows: α isomer, ¹H NMR (500 MHz, Methanol-*d*₄) δ 5.98 (d, *J* = 1.8 Hz, 1H), 4.37 (dd, *J* = 11.9, 2.2 Hz, 1H), 4.29 (dd, *J* = 4.9, 1.9 Hz, 1H), 4.25 (dd, *J* = 11.9, 7.0 Hz, 1H), 4.00 - 3.96 (m, 2H), 3.94 (d, *J* = 15.9 Hz, 1H), 3.81 (ddd, *J* = 9.5, 6.9, 2.2 Hz, 1H), 3.57 (t, *J* = 9.7 Hz, 1H), 2.39 (t, *J* = 7.2 Hz, 2H), 2.31 (t, *J* = 7.3 Hz, 2H), 1.68 (q, *J* = 7.3 Hz, 2H), 1.63 (q, *J* = 7.4 Hz, 2H), 0.99 (t, *J* = 7.4 Hz, 3H), 0.94 (t, *J* = 7.4 Hz, 3H). ¹³C NMR (126 MHz, Methanol-*d*₄) δ 175.0, 172.6, 170.7, 92.9, 73.8, 69.9, 68.3, 64.4, 53.3, 52.4, 36.63, 36.59, 19.2, 19.1, 13.72, 13.66. β isomer, ¹H NMR (500 MHz, Methanol-*d*₄) δ 5.79 (d, *J* = 1.8 Hz, 1H), 4.56 (dd, *J* = 4.6, 1.8 Hz, 1H), 4.39 (dd, *J* = 11.9, 2.3 Hz, 1H), 4.32 (dd, *J* = 12.0, 7.1 Hz, 1H), 3.97 (s, 1H), 3.97 (s, 1H), 3.79 (dd, *J* = 9.4, 4.5 Hz, 1H), 3.59 (ddd, *J* = 9.6, 7.1, 2.4 Hz, 1H), 3.45 (t, *J* = 9.4 Hz, 1H), 2.35 - 2.32 (m, 2H), 2.32 - 2.28 (m, 2H), 1.63 (dh, *J* = 11.7, 7.4 Hz, 4H), 0.94 (t, *J* = 7.4 Hz, 6H), 0.93 (t, *J* = 7.4 Hz, 3H). ¹³C NMR (126 MHz, Methanol-*d*₄) δ 175.0, 172.6, 171.0, 92.7, 76.7, 72.8, 68.6, 64.4, 53.2, 52.5, 36.6, 36.5, 19.2, 18.8, 13.7, 13.6. HRMS (ESI) theoretical molecular weight: C₁₆H₂₇N₄O₈ [M+H]⁺ 403.18289, detected molecular weight: 403.18199.

**3n. 3n** was synthesized from **3i** in two steps, and the synthesis method is similar to that of **3l** in Example 4. The two-step total yield was 83%. α/β, 1.0/0.33.

The NMR detection data of compound **3n** were as follows: ¹H NMR (600 MHz, D₂O) δ 5.94 (d, *J* = 1.8 Hz, 1H, α-H-1), 5.86 (d, *J* = 1.8 Hz, 0.33H, β-H-1), 4.63 (dd, *J* = 4.6, 1.8 Hz, 0.33H, β-H-2), 4.44 (m, 2.33H, β-H-6a +α-H-2 +α-H-6a), 4.35 - 4.31 (m, 0.33H, β-H-6b), 4.28 (dd, *J* = 12.2, 6.3 Hz, 1H, α-H-6b), 4.14 (dd, *J* = 9.8, 4.9 Hz, 1H, α-H-3), 3.98 - 3.92 (m, 1.33H, α-H-5-a+β-H-3), 3.81 - 3.70 (m, 1.33H, β-H-5+α-H-4), 3.63 (t, *J* = 9.8 Hz, 0.33H, β-H-4), 2.67 - 2.35 (m, 11.97H, COCH₂CH₂CCH+ 2 × COCH₂CH₃), 1.11 (m, 7.98H, 2 × COCH₂CH₃). ¹³C NMR (151 MHz, D₂O) δ 177.29 (β-COCH₂CH₃-6), 177.26 (α-COCH₂CH₃-6), 175.91 (β-NHCO), 175.44 (α-NHCO), 175.18 (α-COCH₂CH₃-1), 174.95 (β-COCH₂CH₃-1), 92.22 (α-C-1), 91.77 (β-C-1), 83.58, 83.35, 74.87 (β-C-5), 72.04 (α-C-5), 70.90, 70.22, 70.11, 68.42 (α-C-3), 66.84 (β-C-4), 66.67 (α-C-4), 63.31 (α-C-6), 63.29 (β-C-5), 51.77 (β-C-2), 51.63, 34.32, 34.08, 27.23, 27.21, 27.19, 27.15, 14.48, 14.43, 8.33, 8.29, 8.16, 7.97.

**3o. 3o** was synthesized from **3j** in two steps. The synthesis method was similar to that **of 3l** in Example 4. The two-step total yield was 79%. α/β, 1.0/0.15.

The NMR detection data of compound **3o** were as follows: ¹H NMR (500 MHz, D₂O) δ 6.02 (s, 1.0H, α-H-1), 5.88 (d, *J* = 1.4Hz, 0.15 H, β-H-1), 4.78 - 4.69 (m, 2.30H, NHCOOCH₂CCH), 4.49 (m, 1.15H, α-H-6a+β-H-6a), 4.37 - 4.26 (m, 1.30H, β-H-2+α-H-6b+β-H-6b), 4.21 (d, *J* = 3.6 Hz, 1.0H, α-H-2), 4.14 (dd, *J* = 9.7, 4.7 Hz, 1.0H, α-H-3), 3.97 (ddd, *J* = 8.7, 6.5, 2.1 Hz, 1.15H, α-H-5+β-H-3), 3.78 (ddd, *J* = 9.2, 6.7, 2.1 Hz, 0.15H, β-H-5), 3.69 (t, *J* = 9.9 Hz, 1.0H, α-H-4), 3.60 (t, *J* = 9.8 Hz, 0.15H, β-H-4), 3.10 - 2.93 (m, 1.15 H, NHCOOCH₂CCH), 2.57 - 2.43 (m, 4.6H, 2 × CH₃CH₂COO), 1.20 - 1.12 (m, 6.9H, 2 × CH₃CH₂COO). ¹³C NMR (126 MHz, D₂O) δ 177.40 (β-CH₃CH₂COO-6), 177.38 (α-CH₃CH₂COO-6), 175.25 (α-CH₃CH₂COO-1), 174.98 (β-CH₃CH₂COO-1), 158.33 (β-NHCOOCH₂CCH), 157.61 (α-NHCOOCH₂CCH), 92.62 (α-C-1), 92.04 (β-C-1), 78.59 (β-NHCOOCH₂CCH), 78.50 (α-NHCOOCH₂CCH), 75.93 (α-NHCOOCH₂CCH), 75.87 (β-NHCOOCH₂CCH), 74.92 (β-C-5), 72.21 (α-C-5), 71.00 (β-C-3), 68.73 (α-C-3), 66.98 (β-C-4), 66.82 (α-C-4), 63.53 (α-C-6), 63.46 (β-C-6), 54.05 (β-C-2), 53.43 (α-C-2), 53.18 (NHCOOCH₂CCH), 27.35 (CH₃CH₂COO), 27.30 (CH₃CH₂COO), 27.26 (CH₃CH₂COO), 8.45 (CH₃CH₂COO), 8.41 (CH₃CH₂OCOO), 8.30 (CH₃CH₂COO), 8.13 (CH₃CH₂COO).

### Example 5: In vitro reaction of 1,6-Ac₂GalNAz, 1,6-Pr₂GalNAz, 1,6-Bu₂GalNAz, 6-Ac₂GlcNAz, 1,6-Pr₂GlcNAz, 1,6-Bu₂GlcNAz, 1,6-Ac₂ManNAz, 1,6-Pr₂ManNAz, and 1,6-Bu₂ManNAz with proteins.

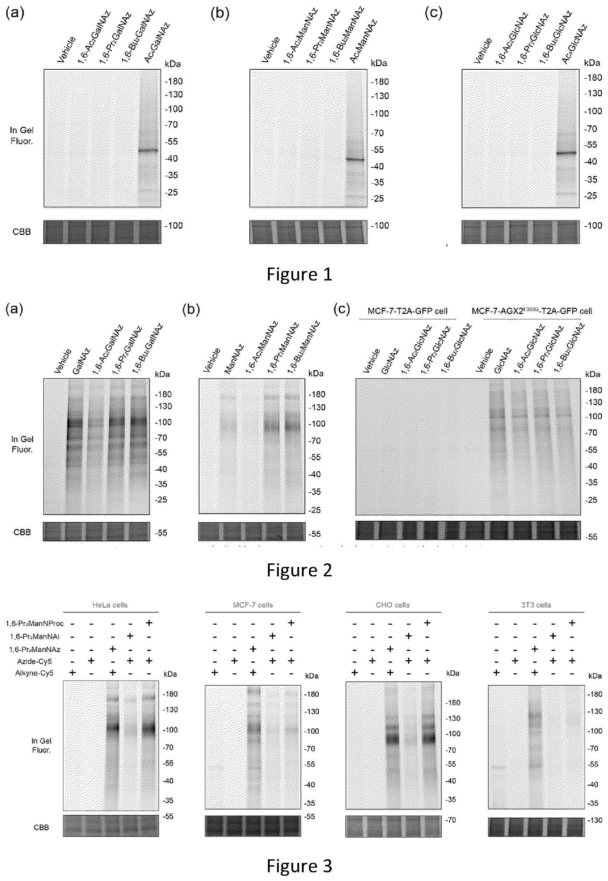

In order to verify that nine partially acylated unnatural sugars, including 1,6-Ac₂GalNAz, 1,6-Pr₂GalNAz, 1,6-Bu₂GalNAz, 6-Ac₂GlcNAz, 1,6-Pr₂GlcNAz, 1,6-Bu₂GlcNAz, 1,6-Ac₂ManNAz, 1,6-Pr₂ManNAz, and 1,6-Bu₂ManNAz, could not spontaneously react with protein cysteine, per-O-acetylated unnatural sugars (Ac₄GalNAz, Ac₄GlcNAz, and Ac₄ManNAz) were used as controls and the above partially acylated unnatural sugars were incubated with protein cell lysates or single protein of HeLa cells, and the azide signal on the protein was detected by electrophoresis gel after reaction at 37 °C for 2h. The reaction results are shown in Figure 1.

**Example 6:** 1,6-Ac₂GalNAz, 1,6-Pr₂GalNAz, 1,6-Bu₂GalNAz, 6-Ac₂GlcNAz, 1,6-Pr₂GlcNAz, 1,6-Bu₂GlcNAz, 1,6-Ac₂ManNAz, 1,6-Pr₂ManNAz, and 1,6-Bu₂ManNAz were used for metabolic glycan labeling effects in living cells.

In order to verify the metabolic labeling effect of the above-mentioned 1,6-bis-acylated unnatural sugars in living cells, HeLa cells were metabolically labeled with the above-mentioned different compounds. The specific steps are as follows:
200 µM of 1,6-bis-acylated unnatural sugar and 2 mM of sugar without protecting groups were added to a culture medium of HeLa cells for culture, and then bioorthogonal reaction was carried out with Cy5 fluorescently labeled substances, and in-gel fluorescence detection was performed on the differently treated HeLa cells. The detection results are shown in Figure 2 (CBB in Figure 3 represents Coomassie Brilliant Blue staining to show the loading amount control).

As can be seen from Figure 2, compared with GalNAz or ManNAz, the labeling efficiency of 200 µM of 1,6-Pr₂GalNAz or 1,6-Pr₂ManNAz is close to or slightly stronger than 2 mM of sugar without protecting groups. Compared with 1,6-Pr₂GalNAz or 1,6-Pr₂ManNAz, the labeling efficiency of 1,6-Bu₂GalNAz or 1,6-Bu₂ManNAz was comparable, but the labeling efficiency of 1,6-Ac₂GalNAz or 1,6-Ac₂ManNAz was much lower (Figures 2 a and b). For MCF-7 (MCF7-T2A-GFP) cells expressing GFP, neither GlcNAz nor 1,6-bis-acylated GlcNAz treatment resulted in obvious labeling (Figure 2c). By overexpressing the mutant enzyme AGX2F383G of AGX2, which can convert GlcNAz-P- into UDP-GlcNAz, GIcNAz and its three corresponding 1,6-bis-acylated derivatives all produced obvious labeling in MCF7-AGX2F383G-T2A-GFP cells (Figure 2c). The above data well illustrate that 1,6-bis-acylated azidosugars can be metabolically labeled at much lower concentrations than unprotected azidosugars.

### Example 7: 1,6-Pr₂ManNAz, 1,6-Pr₂ManNAI and 1,6-Pr₂ManNProc were used for metabolic labeling in living cells.

After different cells were metabolically labeled with 200 µM of 1,6-Pr₂ManNAz, 1,6-Pr₂ManNAl and 1,6-Pr₂ManNProc for 48 hours, the cells were lysed and the probes labeled with Cy5 fluorescence were reacted with glycoproteins with orthogonal groups in the lysate to perform bioorthogonal reactions. After SDS-PAGE separation, in-gel fluorescence analysis and detection was performed. The results are shown in Figure 3 (CBB in Figure 3 represents Coomassie Brilliant Blue staining to show the loading amount control).

### Example 8: 1,6-Pr₂GalNAz and 1,6-Pr₂ManNAz were used for metabolic labeling in vivo in mice.

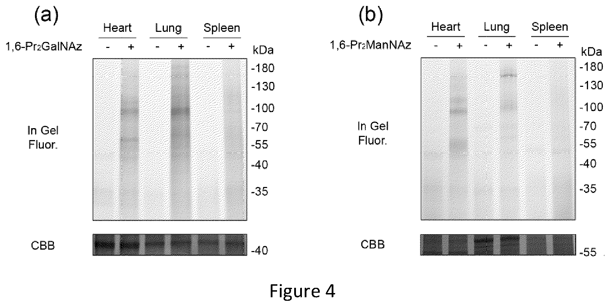

In the in vivo mouse experiment, B6D2F1/J mice were intraperitoneally injected with 1,6-Pr₂GalNAz or 1,6-Pr₂ManNAz (both at a concentration of 500 mg/kg) every day for 3 or 7 consecutive days. The mice were euthanized on the 4th or 8th day, and different organs were selected for homogenization. The azide-modified glycoproteins were click-labeled and separated by SDS-PAGE. In-gel fluorescence analysis showed that glycoproteins from the heart, lung, and spleen could be well labeled, indicating that 1,6-Pr₂GalNAz and 1,6-Pr₂ManNAz can be well used for in vivo metabolic labeling (Figure 4).

It should be particularly pointed out that the various components or steps in the above-mentioned embodiments can be cross-linked, replaced, added, or deleted. Therefore, the combinations formed by these reasonable permutations and combinations should also fall within the scope of protection of the present invention, and the scope of protection of the present invention should not be limited to the embodiments.

The above are exemplary embodiments disclosed in the present invention. The order in which the above embodiments of the present invention are disclosed is only for description and does not represent the advantages or disadvantages of the embodiments. However, it should be noted that the discussion of any of the above embodiments is only exemplary and is not intended to imply that the scope of the disclosure of the embodiments of the present invention (including the claims) is limited to these examples. Various changes and modifications may be made without departing from the scope defined by the claims. The functions, steps and/or actions of the method claims in accordance with the disclosed embodiments described herein do not need to be performed in any particular order. Furthermore, although elements disclosed in the embodiments of the present invention may be described or claimed in individual form, they may also be understood to be in the plural unless explicitly limited to the singular.

Those skilled in the art should understand that the discussion of any of the above embodiments is merely illustrative and is not intended to imply that the scope of the disclosure of the embodiments of the present invention (including the claims) is limited to these examples. Based on the concept of the embodiments of the present invention, the technical features in the above embodiments or different embodiments may also be combined, and there are many other variations of different aspects of the embodiments of the present invention as described above, which are not provided in detail for the sake of simplicity. Therefore, any omission, modification, equivalent substitution, improvement, etc. made within the spirit and principle of the embodiments of the present invention should be included in the protection scope of the embodiments of the present invention.

## Claims

1. A method for synthesizing an unnatural sugar, **characterized by** comprising: subjecting hydroxyl groups of an amino sugar to per-trimethylsilane protection at room temperature, selectively exposing amino groups on the sugar, and coupling and converting the amino groups at room temperature to obtain a per-trimethylsilane-protected unnatural sugar with orthogonal groups.

2. The method according to claim 1, **characterized in that**, trimethylsilane protecting groups are removed from the per-trimethylsilane-protected unnatural sugar with orthogonal groups to obtain an unnatural sugar without protecting groups.

3. The method according to claim 2, **characterized in that**, the unnatural sugar without protecting groups is any one of GalNAz, GlcNAz, ManNAz, GalNAl, GlcNAl and ManNAl;
wherein, during the synthesis of GalNAz, GlcNAz, and ManNAz, the trimethylsilane protecting groups are removed by using a hydrogen ion exchange resin, and a final product is precipitated from a solvent; and
during the synthesis of ManNAl, the trimethylsilane protecting groups are removed by silica gel column chromatography purification.

4. The method according to claim 1, **characterized in that**, the hydroxyl groups at positions 1 and 6 of the per-trimethylsilane-protected unnatural sugar with orthogonal groups are protected by hydrophobic groups to obtain a partially acylated unnatural sugar.

5. The method according to claim 4, **characterized in that**, the hydrophobic groups are any one of acetyl, propionyl, butyryl and valeryl.

6. The method according to claim 4, **characterized in that**, for galactose-type and glucose-type per-trimethylsilane-protected unnatural sugar with orthogonal groups, the trimethylsilane protecting groups at positions 1 and 6 of the per-trimethylsilane-protected unnatural sugar are exchanged into corresponding ester bonds by using pyridine as a solvent and adding four equivalents of carboxylic acid and a large excess of corresponding acid anhydride, and the trimethylsilane protecting groups at positions 3 and 4 are removed by using a hydrogen ion exchange resin to obtain a 1,6-bis-acylated unnatural sugar; and
for a mannose-type per-trimethylsilane-protected unnatural sugar with orthogonal groups, the trimethylsilane protecting groups at positions 1 and 6 are selectively and simultaneously removed by adding two equivalents of ammonium acetate to a mixed solvent of dichloromethane and methanol, and the positions 1 and 6 are protected with ester bonds in pyridine and the trimethylsilane protecting groups at positions 3 and 4 of the sugar are then removed to obtain a 1,6-bis-acylated mannose-type unnatural sugar derivative.

7. The method according to claim 1, **characterized in that**, the orthogonal groups of the per-trimethylsilane-protected unnatural sugar with orthogonal groups are any one of azide groups, terminal alkyne groups, terminal alkene groups, cyclopropene groups, trans-cyclooctene groups, or cyclooctyne groups.

8. A partially acylated unnatural sugar for metabolic labeling, **characterized by** being any one of 1,6-Ac₂GalNAz, 1,6-Pr₂GalNAz, 1,6-Bu₂GalNAz, 1,6-Ac₂GlcNAz, 1,6-Pr₂GlcNAz, 1,6-Bu₂GlcNAz, 1,6-Ac₂ManNAz, 1,6-Pr₂ManNAz, 1,6-Bu₂ManNAz, 1,6-Pr₂ManNAl and 1,6-Pr₂ManNProc;
preferably, the partially acylated unnatural sugar for metabolic labeling being prepared by the method according to any one of claims 1-7.

9. A metabolic glycan labeling reagent kit, **characterized by** comprising the partially acylated unnatural sugar according to claim 8.

10. Use of the partially acylated unnatural sugar according to claim 8 in metabolic glycan labeling reagent kit, for labeling metabolic glycan in any one of HeLa cells, 3T3 cells, CHO cells and MCF-7 cells.
